(19) Europäisches Patentamt
European Patent Office
Office européen des brevets

(11) Veröffentlichungsnummer: **0 195 954**
**A2**

(12) # EUROPÄISCHE PATENTANMELDUNG

(21) Anmeldenummer: **86102716.7**

(51) Int. Cl.⁴: **C 07 D 263/14**

(22) Anmeldetag: **01.03.86**

(30) Priorität: **29.03.85 DE 3511403**

(43) Veröffentlichungstag der Anmeldung: **01.10.86**
**Patentblatt 86/40**

(84) Benannte Vertragsstaaten: **CH DE FR GB LI NL**

(71) Anmelder: **HÜLS AKTIENGESELLSCHAFT,**
**Patentabteilung / PB 15 - Postfach 13 20,**
**D-4370 Marl 1 (DE)**

(72) Erfinder: **Brockhaus, Rudolf, Dr., Holsteiner Strasse 19,**
**D-4370 Marl (DE)**
Erfinder: **Franke, Hans-Jürgen, Freiheitstrasse 13,**
**D-4270 Dorsten (DE)**

(54) **2(1-Hydroxy-1-methyl-ethyl)-5,5-dimethyl-delta 3-oxazolin und Verfahren zu seiner Herstellung.**

(57) 2(1-Hydroxy-1-methyl-ethyl)-5,5-dimethyl-Δ3-Oxazolin der Formel

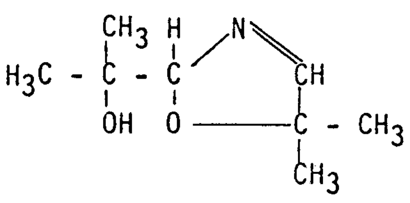

Zur Herstellung von 2(1-Hydroxy-1-methyl-ethyl)-5,5-dimethyl-Δ3-Oxazolin setzt man α-Hydroxyisobutyraldehyd mit Ammoniak oder wäßriger Ammoniaklösung bzw. dessen Acetal (Epoxid) mit wäßriger Ammoniaklösung bei Temperaturen von 20 bis 100°C um. Bevorzugt setzt man konzentrierte wäßrige Ammoniaklösung ein.

EP 0 195 954 A2

## 2(1-Hydroxy-1-methyl-ethyl)-5,5-dimethyl-Δ3-oxazolin und Verfahren zu seiner Herstellung

Die Verbindung 2(1-Hydroxy-1-methyl-ethyl)-5,5-dimethyl-Δ3-oxazolin ist eine neue Verbindung, die interessante chemische Eigenschaften hat und daher als Zwischenprodukt oder Monomerprodukt wertvoll ist.

Die Verbindung ist besonders an der Doppelbindung reaktionsfähig; auch die OH-Gruppe eignet sich für Umsetzungen. Oxazoline werden u. a. als Co-polymere eingesetzt und erlauben es, die anwendungstechnischen Eigenschaften von Kunststoffen gezielt zu verändern.

Für die Herstellung dieser neuen Verbindung wurde ein besonders einfacher Syntheseweg gefunden. α-Hydroxyisobutyraldehyd (II) oder dessen Vorprodukt, das Acetal (Epoxid) (I), werden mit wäßriger Ammoniaklösung zu dem Oxazolin umgesetzt. Der α-Hydroxyisobutyraldehyd kann auch mit wasserfreiem Ammoniak zum Oxazolinderivat umgesetzt werden. Die Reaktion verläuft einstufig, die als Zwischenstufen anzunehmenden Produkte $NH_3$-Aldehyd-Addukt (III) und die Schiff'sche Base treten nicht in Erscheinung. Die Oxazolinverbindung bildet sich sofort und kann z. B. destillativ rein gewonnen werden. Eine Rückreaktion wurde nicht beobachtet, die Oxazolinverbindung ist thermisch stabil. Die cyclische Struktur wurde durch Kernresonanzspektroskopie nachgewiesen.

Die Formel für den skizzierten Weg sind wie folgt:

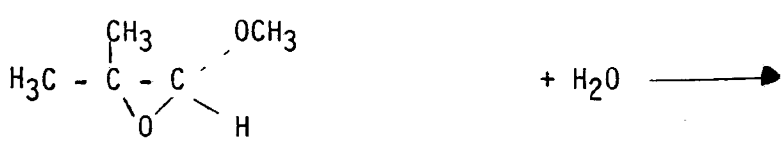

"Acetalepoxid" (I)

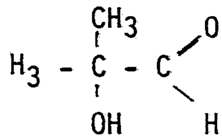

$+ CH_3OH$

α-Hydroxyisobutyraldehyd (II)

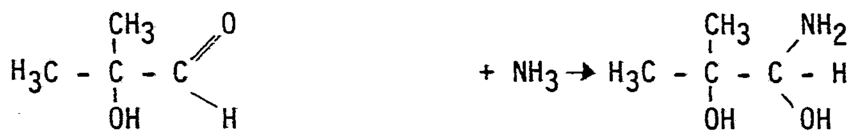


(II)

Aldehyd-$NH_3$-Addukt (III)

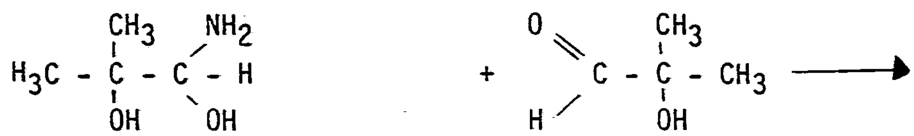


(III)

(II)

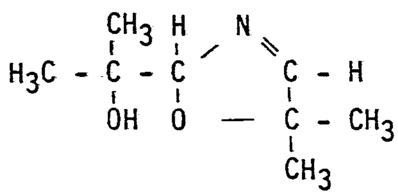

$+ 2\ H_2O$

cyclisches Imin bzw. 2(1-Hydroxy-1-methyl-ethyl)-5,5-dimethyl-Δ3-Oxazolin (IV)

Das Wasser, das für die Hydrolyse des Epoxids benötigt wird, wird mit der wäßrigen Ammoniaklösung zugesetzt. Wasserfreies Ammoniak reagiert unter den Reaktionsbedingungen nicht mit dem Epoxid. Das Epoxid reagiert nur mit wäßriger Ammoniaklösung zum Oxazolinderivat. α-Hydroxyisobutyraldehyd setzt sich mit wäßriger Ammoniaklösung und auch mit wasserfreiem Ammoniak zum

Oxazolinderivat um. Vorzugsweise setzt man konzentrierte wäßrige Ammoniaklösung ein. Die Umsetzung erfolgt bei Temperaturen von 20 bis 100 °C, vorzugsweise bei 30 bis 60 °C, insbesondere bei 35 bis 45 °C. Die Umsetzung erfolgt im allgemeinen bei Normaldruck, sie kann aber auch bei Über- und Unterdruck durchgeführt werden. Die Reaktion kann kontinuierlich und diskontinuierlich durchgeführt werden. Ammoniak bzw. die wäßrige Ammoniaklösung kann man in äquimolekularen Mengen, aber auch im Über- oder Unterschuß einsetzen.

An vier Durchführungsbeispielen wird das Verfahren demonstriert:

1. Umsatz α-Hydroxyisobutyraldehyd mit wäßriger Ammoniaklösung
2. Umsatz Epoxid gereinigt mit wäßriger Ammoniaklösung
3. Umsatz Epoxid roh mit wäßriger Ammoniaklösung
4. Umsatz α-Hydroxyisobutyraldehyd mit wasserfreiem Ammoniak

Die Synthese ist sehr variabel und die Substanz läßt sich auch unter ähnlichen Versuchsbedingungen glatt herstellen, so daß die Versuche nicht alle dem Fachmann geläufigen Synthesevarianten offenlegen.

Beispiel 1

Herstellung der Oxazolinverbindung (2(1-Hydroxy-1-methyl)-5,5-dimethyl-Δ3-oxazolin) aus α-Hydroxyisobutyraldehyd (α-H-IBA)

In einem 2 l-Rührgefäß legt man 430 g wäßrige Ammoniaklösung (23,4 $NH_3$ ≚ 5,92 Mol) bei ca. 20 °C vor. Dazu gibt man innerhalb von ca. 15 Minuten unter gleichzeitiger Erwärmung bis auf ~50 °C 550 g wäßrigen α-H-IBA (15,2 % $H_2O$, 79,9 % α-H-IBA monomer = 4,99 Mol) und läßt anschließend ca. 15 Minuten bei 50 °C nachreagieren.

Der Reaktionsaustrag enthält 0,8 % α-H-IBA und 36,8 % Imin, was einem α-H-IBA-Umsatz von 98,2 % und einer Oxazolin-Ausbeute von 93,7 % entspricht. Der Schmelzpunkt liegt bei 45 °C. Die Verbindung löst sich gut in Wasser, Methanol oder Aceton. Die Elementaranalyse ist:

| | | | | |
|---|---|---|---|---|
| C ber. 61,15 | H ber. 9,55 | N ber. 8,92 | O ber. 20,38 | (Gew.-%) |
| gef. 61,08 | gef. 9,6 | gef. 8,91 | gef. 20,17 | (Gew.-%) |

Molekulargewicht 157

Beispiel 2

Herstellung der Oxazolinverbindung aus acetalischem Epoxid

In einem 2 l-Rührgefäß mit aufgesetztem Rücklaufkühler legt man 400 g 24,7 %ige wäßrige Ammoniaklösung (5,8 Mol) vor und gibt unter Rühren innerhalb von ca. 120 Minuten 1000 g Epoxid (98 %ig, 9,6 Mol) dazu. Die Temperatur wird durch Kühlung bei ca. 40 °C gehalten. Man läßt ca. 15 Minuten bei 40 bis 60 °C nachreagieren. Der Reaktionsaustrag (1392 g) hat folgende Zusammensetzung:

| | |
|---|---|
| 51,90 % Oxazolinderivat | 0,70 % α-H-IBA |
| 20,90 % Methanol | 0,45 % Epoxid |
| 1,40 % $NH_3$ | 2,20 % α-H-IBA-dimethyl-acetal |
| 21,20 % $H_2O$ | 1,15 % Spurenverbindungen |

Aus der Zusammensetzung des Reaktionsaustrages errechnet sich ein Epoxid-Umsatz von 99,4 % und eine Oxazolin-Ausbeute von 96,5 %.

Zur Reingewinnung des Oxazolins wird der Reaktionsaustrag über eine 30-cm-Kolonne, Füllkörper: 6 x 6 mm Drahtnetzringe, destilliert. Dabei kann der Reaktionsaustrag direkt eingesetzt werden. Eine vorherige Abtrennung des Wassers mittels eines Schleppmittels, z. B. n-Hexan, ist für die Vorlauf-/Hauptlauftrennung vorteilhaft.

Die Destillationsbedingungen nach vorheriger Wasser- und Hexanabtrennung sind:

| | |
|---|---|
| Druck: | ca. 25 mbar |
| Rücklaufverhältnis: | 1 : 10/1 : 3 |
| Vorlauf: | 39 bis 87 °C |
| Hauptlauf: | 87 bis 88 °C |

Unter den genannten Destillationsbedingungen werden ca. 90 % des synthetisierten Oxazolinderivates mit einer Reinheit von = 99,6 % isoliert.

Beispiel 3

Herstellung der Oxazolinverbindung aus Rohepoxid (hergestellt aus oxydiertem Enolether nach der DE-PS 31 09 439)

Als Einsatzprodukt für die Oxazolin-Herstellung, wie beim Beispiel 2, wird ein Enolether-Oxidationsaustrag/Rohepoxid eingesetzt, bei dem durch Andestillieren die Leichtsieder bis einschließlich Enolether abgetrennt sind. Bei fast vollständigem Epoxid-Umsatz (> 99 %) liegt die Oxazolin-Ausbeute, bezogen auf mono- und dimeren Epoxideinsatz, bei 99 %. Der Anteil des monomeren Epoxids am Gesamtepoxid lag bei ca. 86 %.

Beispiel 4

Man setzt äquimolekulare Mengen von α-Hydroxyisobutyraldehyd mit wasserfreiem Ammoniak bei 40 °C um. Man erhält das Oxazolinderivat in praktisch quantitativer Ausbeute.

0195954
O.Z. 4063

Patentansprüche:

1. 2(1-Hydroxy-1-methyl-ethyl)-5,5-dimethyl-Δ 3-Oxazolin der Formel

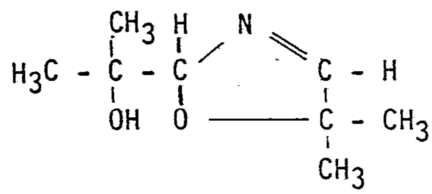

2. Verfahren zur Herstellung von 2(1-Hydroxy-1-methyl-ethyl)-5,5-dimethyl-Δ 3-Oxazolin, dadurch gekennzeichnet, daß man α-Hydroxyisobutyraldehyd mit Ammoniak oder wäßriger Ammoniaklösung bzw. dessen Acetal (Epoxid) mit wäßriger Ammoniaklösung bei Temperaturen von 20 bis 100 °C umsetzt.

3. Verfahren nach Anspruch 2, dadurch gekennzeichnet, daß man die Umsetzung bei Temperaturen von 30 bis 60 °C, vorzugsweise 35 bis 45 °C, durchführt.

4. Verfahren nach den Ansprüchen 2 und 3, dadurch gekennzeichnet, daß man zur Umsetzung konzentrierte waßrige Ammoniaklösung einsetzt.